# FASCICULE DE BREVET EUROPEEN

(11) **EP 2 643 826 B1**
(45) Date de publication et mention de la délivrance du brevet: **24.09.2014**
(21) Numéro de dépôt: 11815550.6
(22) Date de dépôt: 28.12.2011
(51) Int. Cl.: G08B 17/107, G01N 15/06, G01N 21/53

(54) **PROCÉDÉ ET DISPOSITIF DE DÉTECTION DE FUMÉE**
VERFAHREN UND VORRICHTUNG ZUR RAUCHERKENNUNG
METHOD AND DEVICE FOR DETECTING SMOKE

(30) Priorité: 31.12.2010 FR 1005201
(43) Date de publication de la demande: 02.10.2013
(73) Titulaire: Finsécur, 92000 Nanterre (FR)
(72) Inventeur: LEWINER, Jaques, F-92210 Saint Cloud (FR)
(74) Mandataire: Cornuejols, Georges
(86) Numéro de dépôt international: PCT/FR2011/053203
(87) Numéro de publication internationale: WO 2012/089986

(56) Documents cités:
- WO-A1-00/43965
- WO-A2-2007/051170
- FR-A1- 2 438 841
- FR-A1- 2 594 953
- FR-A1- 2 723 233
- FR-A1- 2 746 184
- US-A- 3 823 372
- US-A- 3 932 851
- US-A- 4 225 860
- US-A- 4 469 953
- US-A1- 2004 163 955

## Description

La présente invention est relative à un dispositif et à un procédé de détection de fumée. Elle s'applique, en particulier, à la détection d'un incendie grâce à la présence de fines particules ou aérosols contenus dans les fumées, ce qui permet de réduire les risques d'incendie des locaux où sont placés de tels dispositifs ou où sont mis en oeuvre de tels procédés.

Afin de détecter la présence de fumée, on utilise principalement deux effets physiques, à savoir la diffusion de lumière par la fumée, les poussières ou les aérosols qui lui sont associés ; et la modification, sous l'effet de ces fumées, poussières ou aérosols, de la vitesse de déplacement d'ions entraînés par un champ électrique.

Dans le premier cas, un détecteur optique de fumée, tel que décrit par exemple dans la demande de brevet FR 2 723 233, comprend une chambre comportant des ouvertures propres à permettre à la fumée de pénétrer dans la chambre, une source de lumière susceptible, lorsqu'elle est alimentée, d'émettre dans la chambre un faisceau lumineux, un récepteur de lumière propre à transformer la réception de lumière en un signal électrique de réception, l'intérieur de la chambre étant peu réfléchissant dans le domaine spectral de la lumière émise par la source, les ouvertures étant associées à des chicanes propres à empêcher la pénétration directe dans la chambre de la lumière extérieure et la source et le récepteur étant placés de manière propre à empêcher l'arrivée directe de la lumière émise par la source sur le récepteur.

Ainsi, en l'absence de fumées contenant des particules assez grosses pour diffuser la lumière émise par la source, typiquement de dimensions supérieures à 100 nm, le récepteur ne reçoit pratiquement pas de lumière et ne fournit donc qu'un signal électrique de réception très petit, dû uniquement à la lumière qui pénètre de l'extérieur de la chambre et aux quelques éventuelles réflexions sur les parois intérieures de la chambre de la lumière émise par la source.

Au contraire, la présence de fumées ou d'aérosols, associés à des particules de dimensions supérieures à 100 nm dans le faisceau lumineux, se traduit par l'apparition de faisceaux diffusés dont certains atteignent le récepteur, éventuellement après réflexions sur les parois internes de la chambre, faisant apparaître un signal électrique de réception.

Le dépassement d'un seuil prédéterminé de ce signal permet alors le déclenchement ou la transmission d'une alarme.

Les détecteurs optiques de fumée sont particulièrement sensibles à la fumée visible, c'est-à-dire constituée de grosses particules, mais restent relativement insensibles aux fumées de fines particules donc quasiment invisibles et qui sont émises lors des premières étapes du développement de l'incendie ou dans un feu vif.

En effet, lorsque la fumée est constituée de fines particules trop petites ou en trop faible concentration, la diffusion de lumière est très faible et le récepteur reçoit une quantité de lumière insuffisante pour permettre le franchissement du seuil de déclenchement ou de transmission d'une alarme.

Dans le second cas, un détecteur ionique de fumée comprend une chambre dans laquelle sont disposées deux électrodes de mesure entre lesquelles on crée, ou on amène, des ions de charges qi.

L'application d'une différence de potentiel entre ces électrodes produit un champ électrique E qui exerce une force F = qi x E sur ces ions, ce qui fait apparaître un courant électrique nominal entre les électrodes et dans le circuit extérieur qui les relie, ce courant électrique dépendant en particulier de la quantité d'ions présents dans la chambre, de la différence de potentiel appliquée entre les électrodes de mesure, et de la mobilité des ions.

Des moyens de mesure de ce courant sont en outre prévus, qui fournissent un signal exploitable par des moyens d'exploitation.

Lorsque des particules associées à de la fumée entrent dans la chambre, un certain nombre de ces particules s'attachent à des ions de la chambre sous l'effet des forces électrostatiques créées par ces ions, ce qui réduit leur mobilité et a pour effet de diminuer le courant électrique.

Si la tension appliquée aux électrodes est assez faible, typiquement comprise entre 5 et 30 volts, le courant électrique nominal est également faible, typiquement compris entre 10 pA et 100 µA, et le ralentissement des ions résultant de la présence des particules est de nature à réduire très sensiblement l'amplitude de ce courant.

Les moyens d'exploitation sont agencés de façon à permettre le déclenchement ou la transmission d'une alarme lors du dépassement vers le bas par le courant mesuré d'un seuil prédéterminé.

Les dispositifs à ionisation d'air sont eux plus sensibles aux produits de combustion, émis lors des premiers développements de l'incendie ou lors de feux vifs, dont les dimensions peuvent atteindre des valeurs de l'ordre de quelques dizaines de nm, voire moins, et permettent ainsi de déclencher des alertes de façon plus précoce que les dispositifs optiques, permettant ainsi de limiter les conséquences de ces incendies.

Deux approches ont été utilisées pour créer des ions dans la chambre de mesure : soit en ionisant l'air à l'aide d'une petite source radioactive, tel que décrit par exemple dans la demande de brevet FR 259 49 53, soit par création d'un champ électrique supérieur au champ électrique de claquage de l'air, tel que décrit par exemple dans le brevet US 3 823 372.

La première approche est simple à mettre en oeuvre et peu coûteuse.

On utilise par exemple une source de particules α en Am241 d'activité comprise entre 0,1 et 1 microcurie, ces particules pouvant franchir dans l'air une distance de l'ordre du centimètre et ainsi ioniser le volume traversé.

Toutefois, cette solution, qui permet pourtant une détection précoce des incendies et donc une limitation de leurs conséquences, est de plus en plus contestée, soit par les utilisateurs eux-mêmes qui sont réticents à la multiplication de sources radioactives dans leurs locaux, soit par les services commerciaux des fabricants qui doivent faire face aux réactions négatives de leurs clients, soit encore au niveau des réglementations.

En ce qui concerne la seconde approche, diverses solutions ont été proposées pour ioniser l'air. Ces approches utilisent le fait qu'en appliquant une différence de potentiel supérieure à un certain seuil Vs entre deux électrodes, on peut amorcer un processus de décharge électrique et ainsi créer des ions.

La valeur V_{S} dépend de plusieurs paramètres tels que la nature du gaz entre les électrodes, la pression du gaz qui les sépare, la distance entre les électrodes et leur forme, la présence de poussières ou d'humidité, etc.

Dans l'air, on considère que ce seuil est situé à environ 330 V pour des distances entre électrodes de l'ordre du micromètre, distances trop petites pour être directement utilisées dans un détecteur de fumée, ce qui oblige pour créer cette ionisation à utiliser des tensions de l'ordre de quelques kilovolts.

De telles valeurs ne peuvent toutefois être celles utilisées pour polariser les électrodes de mesure car la vitesse élevée des ions qui en résulterait conduirait à un courant électrique nominal très élevé et à un temps de traversée par ces ions de la chambre de mesure très court.

En conséquence, les modifications de ce courant par la présence des particules associées à la fumée seraient tellement faibles qu'elles seraient difficilement détectables.

Pour contourner cet obstacle, diverses propositions ont été faites pour augmenter le temps d'interaction entre les ions et les particules de fumée.

Une première approche a été d'utiliser une chambre de mesure polarisée par une tension faible dans laquelle sont transférés, au moyen d'un faible courant d'air, des ions produits dans une chambre d'ionisation polarisée par une haute tension, et ainsi avoir un courant nominal faible.

Un exemple d'une telle solution est décrit dans la demande de brevet FR 2 746 184.

Dans une seconde approche, on a introduit des éléments réfléchissants entre les électrodes d'une chambre d'ionisation polarisée par une haute tension, de façon à augmenter le temps d'interaction.

Un exemple d'une telle solution est décrit dans le brevet US 3 932 851.

Ces solutions alternatives mettant en oeuvre des tensions électriques élevées conduisent toutefois à des détecteurs ayant soit des sensibilités de détection relativement faibles du fait même de l'utilisation de hautes tensions, ce qui en réduit fortement l'intérêt, soit à des dispositifs mécaniquement complexes, fragiles et coûteux.

De plus, la réponse de ces détecteurs est influencée également par des paramètres tels que des variations de pression du gaz ambiant ou de la température, ce qui impose l'utilisation conjointe de dispositifs de compensation, tels que par exemple décrits dans le brevet EP-236 223.

Pour ces raisons, ces solutions alternatives n'ont pas connu de grands développements industriels.

Afin de permettre de détecter tant les particules de petite dimension que celles plus grosses, et ainsi de couvrir les risques associés aux différentes phases d'un incendie, il a été proposé, par exemple dans le brevet US 4 469 953, de combiner les dispositifs de détection ionique de fumée bien adaptés à la détection des petites particules et les dispositifs de détection optique de fumée bien adaptés à la détection des plus grosses particules, à l'intérieur d'un même détecteur.

Dans ce cas, afin de permettre la construction de chambres peu réfléchissantes optiquement et blindées du point de vue électromagnétique, tout en étant de faible coût, il a été proposé de réaliser les électrodes ou blindages à l'aide de plastiques conducteurs, plutôt qu'à l'aide de pièces métalliques, comme par exemple décrit dans les brevets US 4 469 953 et 4 225 860.

Bien évidemment la partie ionique de ces détecteurs présente les mêmes inconvénients que les détecteurs, fonctionnant selon le seul mode ionique, tel que précédemment décrit.

Enfin la demande de brevet FR 2438841 divulgue un autre capteur ionique pour incendie ; les demandes de brevet WO2007/051170 et US2004/0163955 décrivent le principe de la diélectrophotorèse appliquée à l'analyse d'échantillons biologiques.

La présente invention vise à remédier à tout ou partie de ces inconvénients.

A cet effet selon un premier aspect, la présente invention vise un détecteur de fumée selon la revendication 1.

Selon un deuxième aspect la présente invention vise un procédé de détection de fumée selon la revendication indépendante de procédé.

Ainsi, une particule de fumée neutre de polarisabilité α acquiert, sous l'effet du champ créé par les moyens de concentration, un moment dipolaire électrique et est soumise à une force proportionnelle au produit de α par le carré du gradient du champ électrique, ce qui a pour effet de l'attirer vers la zone de concentration de gradient élevé, réalisant ainsi-dans cette zone de concentration une concentration des particules à détecter. Le gradient spatial de champ électrique est apte à exercer une force diélectrophorétique sur les particules de fumée de dimension comprise entre 0,01 µm et 10 µm.

De plus, dans la région de concentration des particules de fumée, ces dernières, du fait de la polarisation qu'elles ont acquise, ont tendance à s'agréger et à constituer ainsi des « quasi » grosses particules propres à assurer une bonne diffusion de la lumière reçue de la source de lumière quand bien même les particules de fumée constituantes de l'agrégat ne seraient pas assez grosses, ou en concentration suffisante, pour diffuser par elles-mêmes la lumière.

Les moyens de concentration peuvent comprendre des électrodes propres à créer dans au moins une partie de la zone de détection un champ électrique non-homogène, et/ou un dispositif de focalisation agencé pour focaliser des rayons lumineux, dans la zone de détection pour engendrer des gradients de champ électromagnétique dans la zone de détection.

Dans des modes de réalisation préférés, on a recours à l'une et/ou l'autre des dispositions suivantes :
- le champ électromagnétique non homogène est apte à agréger les particules de fumée dans la zone de concentration pour former des particules de fumée plus grandes,
- les moyens de concentration comprennent deux électrodes propres à créer dans au moins une partie de la zone de détection un champ électrique non-homogène, les électrodes étant agencées de façon à créer dans la zone de détection un gradient spatial de champ électrique lorsqu'une différence de potentiel inférieure au seuil d'ionisation du gaz dans la chambre, est appliquée à ces électrodes,

- l'une des électrodes est constituée par une partie de la chambre elle-même,
- lorsque l'une des électrodes est constituée par une partie de la chambre elle-même, la chambre est constituée par un polymère conducteur,
- lorsque l'une des électrodes est constituée par une partie de la chambre elle-même, l'une des électrodes comporte des aspérités à faible rayon de courbure,
- des moyens sont propres à appliquer aux électrodes des impulsions de tension d'amplitude ΔV et de durée ΔTv,
- les impulsions de tension ont une amplitude comprise entre 2 et 300 V,
- les impulsions de tension ont une amplitude comprise entre 2 et 30 V,
- les électrodes sont constituées ou recouvertes d'un matériau propre à réduire ou empêcher l'adhérence sur leur surface externe de poussières et/ou l'humidité dans la sous-zone de fort gradient de champ électrique,
- les électrodes sont recouvertes d'une couche hydrophobe,
- les électrodes sont recouvertes d'une couche hydrophobe contenant un polymère fluoré,
- les moyens de concentration comprennent un dispositif de focalisation agencé pour focaliser des rayons lumineux, dans la zone de détection pour engendrer des gradients de champ électromagnétique dans la zone de détection. Les rayons lumineux à focaliser peuvent être émis par la source de lumière ou par une deuxième source de lumière distincte de la source de lumière,
- le dispositif de focalisation est agencé pour focaliser des rayons lumineux dans le domaine infrarouge,
- le dispositif de focalisation comprend une lentille, un prisme ou un guide de lumière,
- les moyens d'alimentation de la source fournissent des impulsions de courant d'amplitude ΔI et de durée ΔTi,
- les impulsions de courant ont une durée comprise entre 100 ns et 10 ms,
- les impulsions de courant sont émises à des intervalles de temps compris entre 100 ms et 10s,
- les moyens d'exploitation sont propres à analyser la variation temporelle de l'amplitude du signal électrique,
- les moyens d'exploitation sont propres à comparer l'amplitude du signal à un niveau de référence,
- le niveau de référence est fixé,
- le niveau de référence est déterminé par des moyens,
- les moyens sont agencés pour modifier le niveau de référence en fonction des valeurs du signal électrique mesuré précédemment,
- les moyens sont agencés pour modifier le niveau de référence en fonction des valeurs du signal électrique moyen mesuré précédemment,
- les moyens sont agencés pour modifier le niveau de référence en fonction de la nature du risque d'incendie à surveiller,
- lorsque l'une des électrodes est constituée par une partie de la chambre elle-même, l'autre électrode est située entre l'émetteur et le récepteur et empêche la vision directe de l'émetteur par le récepteur,
- dans le cas précédent, ladite autre électrode a une arête vive dans une direction perpendiculaire à la direction émetteur/récepteur,
- l'une des électrodes est un fil conducteur placé parallèlement à l'axe de propagation de la lumière émise par la source,
- dans le cas précédent, le rayon du fil est compris entre 10 µm et 1 mm,
- le rayon du fil est compris entre 20 µm et 200 µm,
- l'une des électrodes est constituée par l'arête d'un prisme, cette arête étant placée parallèlement à l'axe de propagation de la lumière émise par la source,
- le rayon de courbure de l'arête ci-dessus est compris entre 10 µm et 1 mm,
- le rayon ci-dessus est compris entre 20 µm et 200 µm,
- dans l'un des six cas ci-dessus, l'axe de propagation de la lumière est positionné entre la zone de forte courbure du fil ou de l'arête et la seconde électrode,
- dans le cas ci-dessus, la distance entre les électrodes propres à créer dans au moins une partie de la zone de détection un champ électrique non-homogène est comprise entre 2 et 10 mm.
- la source de lumière est une diode laser,
- l'axe de sensibilité maximale du récepteur de lumière et de son système optique associé et l'axe d'émission maximale de la source de lumière et de son système associé sont agencés de telle sorte que les rayons lumineux issus de la zone de détection forment avec les rayons lumineux arrivant dans la zone de détection en provenance de la source de lumière et de son système associé un angle compris entre 120 et 140 degrés,
- les fenêtres sont recouvertes par des grilles filtrant les particules de dimension supérieures à 1 mm,
- la dimension des orifices constituant les fenêtres est comprise entre 50 µm et 500 µm,
- la dimension des orifices constituant les fenêtres est comprise entre 100 µm et 400 µm,
- les fenêtres sont constituées de feuilles possédant des ouvertures et sont recouvertes au moins sur leur partie extérieure à la chambre par un matériau hydrophobe,
- les impulsions de tension ΔV et les impulsions de courant ΔI sont appliquées simultanément,
- les impulsions de tension ΔV sont appliquées avec un retard ΔT1 après l'émission des impulsions de courant ΔI,
- le retard ΔT1 est compris entre 1 et 60 µs,
- le retard ΔT1 est compris entre 10 et 30 (µs,
- la durée ΔTi de l'impulsion de courant ΔI est comprise entre 20 et 60 µs, la durée ΔTv de l'impulsion de tension ΔV est comprise entre 20 et 60 µs et le retard ΔT1 est compris entre 20 et 60 µs,
- la durée ΔTi de l'impulsion de courant ΔI est de l'ordre de 50 µs, la durée ΔTv de l'impulsion de tension ΔV est de l'ordre de 25 µs et le retard ΔT1 est de l'ordre de 25 µs,
- les impulsions de tension ΔV sont émises avec un retard ΔT2 après l'application des impulsions de courant ΔI,
- le retard ΔT2 est compris entre 1 et 60 µs,
- le retard ΔT1 est compris entre 10 et 30 µs,
- la durée ΔTi de l'impulsion de courant ΔI est comprise entre 20 et 60 µs, la durée ΔTv de l'impulsion de tension ΔV est comprise entre 20 et 60 µs et le retard ΔT2 est compris entre 20 et 60 µs,
- la durée ΔTi de l'impulsion de courant ΔI est de l'ordre de 50 µs, la durée ΔTv de l'impulsion de tension ΔV est de l'ordre de 25 µs et le retard ΔT2 est de l'ordre de 25 µs,
- les impulsions de tension ΔV ne sont émises qu'avec certaines impulsions de courant ΔI,
- la source de lumière est une diode électroluminescente,
- la source de lumière est une diode laser, et
- la lumière émise par la source a une longueur d'onde comprise entre 800 nm et 1000nm.

Ainsi, selon ces modes de réalisation de l'invention, on dispose d'un dispositif simple et peu coûteux qui permet de détecter les fumées à grosses particules comme le ferait un détecteur optique et les fumées à fines particules ou les aérosols comme le ferait un détecteur ionique, et ce, sans utilisation de sources radioactives ou de hautes tensions électriques.

L'invention comprend, mises à part ces dispositions principales, certaines autres dispositions qui s'utilisent de préférence en même temps et dont il sera plus question ci-après.

Dans ce qui suit, on va décrire quelques modes de réalisations préférés de l'invention en se référant aux figures ci-annexées d'une manière bien entendu non limitative.

La figure 1 représente schématiquement un premier mode de réalisation particulier du dispositif de détection de fumée objet de l'invention.

Les figures 2a et 2b décrivent schématiquement l'allure des lignes de champ électrique entre deux électrodes de formes propres à créer une zone de fort gradient de champ selon des modes de réalisation particulier, entre lesquelles se trouvent des particules de fumée, ces particules, bien que neutres, étant soumises à une force dans la direction K.

La figure 2c décrit schématiquement l'effet d'attraction entre les différentes particules sous l'effet de leur polarisation induite.

La figure 3 est une représentation graphique en vue de dessus, de trois exemples de géométrie d'électrodes selon des modes de réalisation particuliers, propres à créer un gradient de champ électrique lors de l'application d'une différence de potentiel entre ces électrodes :
Figure 3a : pointe-cylindre,
Figure 3b : fil-cylindre,
Figure 3c : arête-plan.

La figure 4 est un exemple de représentation temporelle de l'évolution du signal de réception Si lorsqu'on applique une impulsion de courant ΔI à une source de lumière et une impulsion de tension ΔV entre les électrodes selon des modes de réalisation particuliers.

La figure 5 représente schématiquement un détecteur de fumée selon un deuxième mode de réalisation particulier du dispositif objet de l'invention.

La figure 6 représente schématiquement un détecteur de fumée selon un troisième mode de réalisation particulier du dispositif objet de l'invention.

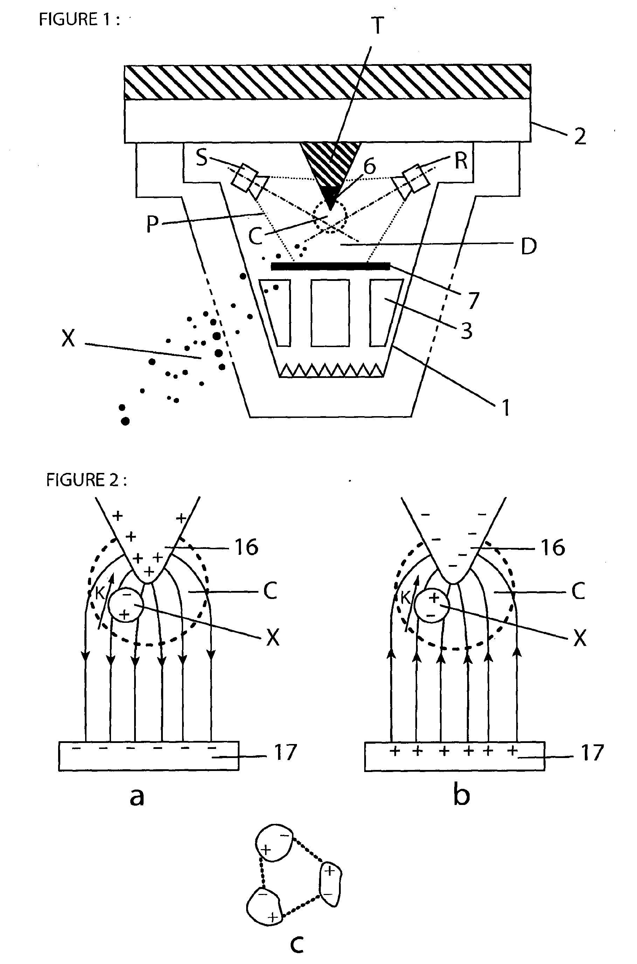

Un détecteur de fumée selon un premier mode de réalisation de l'invention est représenté schématiquement sur la figure 1. Ce détecteur de fumée comprend une chambre 1, percée, d'une façon connue en soi, d'ouvertures 3 pour permettre le passage de l'air et des particules de fumée à contrôler vers une zone de détection D à l'intérieur de la chambre 1.

Ces ouvertures 3 sont associées à des chicanes, permettant de limiter la pénétration de lumière externe dans la chambre. Les parois internes de la chambre 1 sont agencées pour réfléchir au minimum les rayons lumineux.

Les ouvertures 3 peuvent être recouvertes par des grilles filtrant les particules de dimension supérieures à 1 mm. Dans ce mode de réalisation de l'invention, la dimension des orifices constituant les ouvertures est comprise entre 50 µm et 500 µm, et préférentiellement entre 100 µm et 400 µm

Les ouvertures 3 peuvent être constituées de feuilles possédant des ouvertures et sont recouvertes au moins sur leur partie extérieure à la chambre par un matériau hydrophobe.

La chambre contient au moins une source de lumière S, par exemple une diode laser ou une diode électroluminescente, apte à émettre vers la zone de détection D, lorsqu'elle reçoit un courant électrique fourni par des moyens d'alimentation électrique, un faisceau de rayons lumineux P ; et au moins un récepteur de lumière R sensible pour au moins une partie des longueurs d'onde des rayons lumineux émis par la source de lumière S et propre à transformer la réception de lumière en un signal électrique de réception.

Le récepteur R est placé dans une zone d'ombre de la chambre 1 vis-à-vis de la source de lumière S pour empêcher l'arrivée directe de la lumière émise par la source de lumière S sur le récepteur R.

Cette zone d'ombre est créée, dans ce mode de réalisation, à l'aide d'une petite pièce séparatrice ou paroi optique T placée entre la source de lumière S et le récepteur R pour empêcher la lumière émise par la source de la lumière S d'atteindre directement le récepteur R. Ainsi la source de lumière S est positionnée de l'un des côtés de la paroi T et le récepteur R est disposé de l'autre côté de la paroi T de façon telle que des rayons lumineux ne puissent circuler de manière directe de la source de lumière S au récepteur R. La paroi optique T présente deux surfaces opposées crénelées pour réfléchir au minimum les rayons lumineux.

Le récepteur R est agencé pour recevoir, en présence de particules de fumée ou des agrégats de particules de fumée dans la zone de détection D, de la lumière diffusée en provenance de la zone de détection D.

Si aucune fumée n'est présente dans la zone de détection D de la chambre lors de l'émission du faisceau des rayons lumineux P, le récepteur R ne reçoit que les pinceaux lumineux éventuellement réfléchis par les parois intérieures de la chambre 1, ce qui a pour conséquence que le signal électrique fourni par le récepteur est très faible, voire pratiquement nul.

Si au contraire la chambre 1 contient de la fumée dans la zone de détection D, les particules associées X sont susceptibles de provoquer une diffusion de la lumière de telle sorte que le récepteur R peut recevoir certains des rayons ainsi diffusés, ce qui fait apparaître un signal électrique en sortie du détecteur R.

Ce signal est envoyé vers des moyens d'exploitation propres à comparer l'amplitude du signal à un niveau de référence et à en analyser la dépendance temporelle.

Le dépassement par ce signal d'un certain seuil, ou la modification de sa dépendance temporelle également au-delà d'une limite définie permet de déclencher ou de transmettre une alarme.

L'amplitude de ce signal est d'autant plus élevée que la densité de la fumée considérée est elle-même plus élevée et que les particules associées sont grosses.

L'inventeur a constaté qu'il était possible, en utilisant des propriétés remarquables de la matière et de son comportement dans certains champs électriques à géométrie très particulière, de concentrer les particules de fumée dans certaines régions de la chambre 1 dites région de concentration C et de créer des agrégats de fines particules, propres à diffuser la lumière émise par la source de lumière S quand bien même les fines particules de fumée qui constituent les agrégats n'auraient pas d'effet direct décelable sur cette lumière.

Pour cela on place à l'intérieur de la chambre 1 des moyens de concentration (6, 7, 16, 17, 26, 27, 36, 37, 46 et 47 dans les figures) propres à créer, dans la zone de détection D, à l'intérieur de la chambre 1 au moins une sous zone dite à fort gradient dans laquelle est créé un fort gradient de champ électrique ou électromagnétique. En effet ces moyens de concentration sont configurés pour créer dans la zone de détection D de la chambre 1 un champ électrique non-homogène apte, en présence de fumée, à polariser des particules de fumée qui pénètrent dans la zone de détection D, le champ électrique non-homogène ayant un gradient spatial de champ électrique apte à exercer une force diélectrophorétique sur les particules de fumée présentes dans le champ électrique non-homogène, cette force diélectrophorétique étant apte à entraîner les particules de fumée polarisées dans une sous-zone de concentration C qui correspond à la sous zone dite à fort gradient, dans la zone de détection D et les unes vers les autres, ce qui a pour effet de constituer des agrégats.

Selon le premier mode de réalisation de l'invention illustré en figure 1 ces moyens de concentration sont constitués de deux électrodes, 6 et 7, disposées dans la chambre dans la zone de détection D et agencés de manière à créer, lors de l'application d'une tension V entre ces électrodes 6 et 7, un champ électrique possédant un fort gradient de champ dans une région de concentration C de la zone de détection D.

L'application de la tension V entre les électrodes 6 et 7 crée un gradient de champ apte à engendrer dans la région de concentration C un effet de diélectrophorèse sur les particules de fumée fines, de dimension comprise entre 0,01 µm et 10 µm. On rappelle que la diélectrophorèse est un effet qui crée une force sur une particule diélectrique lorsqu'elle est soumise à un champ électrique non-homogène de fort gradient. Ainsi, une particule de fumée neutre de polarisabilité α acquiert, sous l'effet du champ électrique, un moment dipolaire électrique et est soumise à une force proportionnelle au produit de α par le carré du gradient du champ électrique, ce qui a pour effet de l'attirer, quelle que soit la polarité de la tension appliquée, vers la zone de gradient élevée, réalisant ainsi dans cette zone de gradient élevé, dite région de concentration C, une concentration des particules à détecter.

De plus, dans la région de concentration C des particules, ces dernières, du fait de la polarisation qu'elles ont acquise sous l'effet du champ électrique, ont tendance à s'agréger et à constituer ainsi des « quasi » grosses particules propres à assurer une bonne diffusion de la lumière reçue de la source de lumière S, dans la zone de détection D quand bien même les particules constituantes de l'agrégat ne seraient pas assez grosses, ou en concentration suffisante, pour diffuser la lumière.

Les figures 2a et 2b illustrent schématiquement l'allure des lignes de champ électrique entre deux électrodes 16 et 17 qui créent, lorsqu'une tension V est appliquée entre les deux électrodes, une zone de fort gradient de champ, entre lesquelles se trouvent des particules de fumée soumises à une force dans la direction K.

La figure 2c illustre schématiquement l'effet d'attraction entre les différentes particules de fumée sous l'effet de leur polarisation induite.

De nombreuses configurations géométriques des électrodes sont possibles afin d'obtenir des gradients de champ électrique élevé : dans l'exemple représenté sur la figure 1, on applique une tension entre une pointe 6, par exemple métallique et une surface plane conductrice 7, par exemple en plastique conducteur.

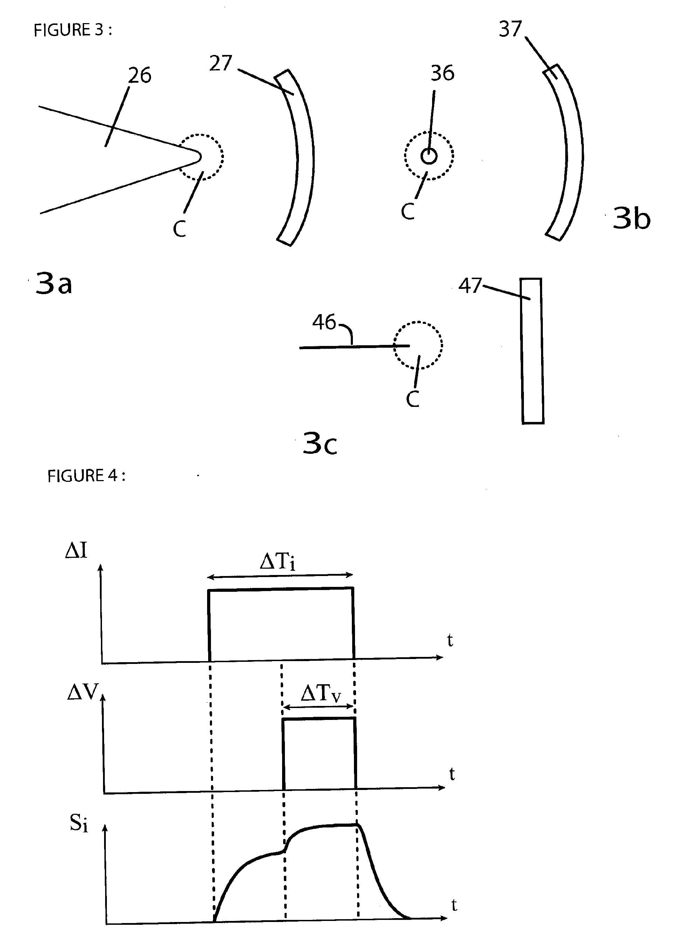

Il est également possible d'obtenir des gradients de champ électrique qui engendrent l'effet diélectrophorétique en utilisant de la même façon une pointe conductrice 16 et une surface conductrice 17 comme représenté sur la figure 2a ou 2b ou, comme représenté sur la figure 3a, une pointe conductrice 26 et une surface cylindrique 27 ou, comme représenté sur la figure 3b, un segment de fil cylindrique conducteur 36 et une portion d'arc 37 d'une surface cylindrique conductrice, ou comme représenté sur la figure 3c l'arête d'un plan conducteur 46 et un plan conducteur 47 ou selon tout autre méthode connue par ailleurs.

Si des particules de fumée chargées sont également présentes, elles sont quant à elles directement entraînées par la force électrique F, F étant égale au produit de q, la charge électrique de la particule, par E, le champ électrique généré par l'application de la tension entre les électrodes (6, 7, 16, 17, 26, 27, 36, 37, 46 et 47).

De cette manière, il est possible de concentrer les particules de fumée dans une région de concentration C prédéterminée du détecteur de telle sorte que le rayon lumineux en provenance de la source de lumière S et incident dans cette région de concentration C est diffusé vers le récepteur R par cette concentration élevée de particules de fumée, ce qui a l'effet d'augmenter la sensibilité du détecteur.

Dans des modes de réalisation de l'invention, l'une des électrodes est constituée par une partie de la chambre elle-même. Lorsque l'une des électrodes est constituée par une partie de la chambre elle-même, la chambre peut être constituée par un polymère conducteur. Lorsque l'une des électrodes est constituée par une partie de la chambre elle-même, l'une des électrodes peut comporter des aspérités à faible rayon de courbure. Lorsque l'une des électrodes 7 est constituée par une partie de la chambre elle-même, l'autre électrode est située entre l'émetteur et le récepteur pour empêcher la vision directe de l'émetteur par le récepteur. Ladite autre électrode peut avoir une arête vive dans une direction perpendiculaire à la direction source de lumière/récepteur.

Dans des modes de réalisation, l'une des électrodes comprend un fil conducteur placé parallèlement à l'axe de propagation de la lumière émise par la source de lumière. Le rayon du fil peut être compris entre 15 µm et 1 mm, et préférentiellement entre 20 µm et 400 µm.

Dans des modes de réalisation, l'une des électrodes est constituée par l'arête d'un prisme, cette arête étant placée parallèlement à l'axe de propagation de la lumière émise par la source. Le rayon de courbure de l'arête ci-dessus peut être compris entre 15 µm et 1 mm, et préférentiellement entre 100 µm et 500 µm. L'axe de propagation de la lumière de la source de lumière S est positionné entre la zone de forte courbure du fil ou de l'arête et la seconde électrode.

Les moyens d'alimentation peuvent être prévus pour appliquer aux électrodes (6, 7, 16, 17, 26, 27, 36, 37, 46 et 47) des impulsions de tension d'amplitude ΔV et de durée ΔTv. Les impulsions de tension peuvent avoir une amplitude comprise entre 2 et 300 Volts, et préférentiellement comprise entre 2 et 30 Volts.

Dans des modes de réalisation de l'invention, les électrodes peuvent être constituées ou recouvertes d'un matériau propre à réduire ou empêcher l'adhérence sur leur surface externe de poussières et/ou l'humidité dans la sous-zone de concentration C de fort gradient de champ électrique. Les électrodes peuvent être recouvertes d'une couche hydrophobe, et préférentiellement d'une couche hydrophobe contenant un polymère fluoré.

Les moyens d'alimentation de la source de lumière S peuvent être prévus pour fournir à la source de lumière S des impulsions de courant d'amplitude ΔI et de durée ΔTi. Les impulsions de courant peuvent avoir une durée ΔTi comprise entre 100 ns et 1 ms. Les impulsions de courant peuvent être émises à des intervalles de temps compris entre 100 ms et 10 s.

Les impulsions de tension fournies aux électrodes et les impulsions de courant fournies à la source de lumière S peuvent être appliquées simultanément, ou les impulsions de tension peuvent être appliquées avec un retard ΔT1 après l'émission des impulsions de courant. Le retard ΔT1 peut être compris entre 1 µs et 60 µs, et préférentiellement entre 10 et 30 µs.

La durée ΔTi de l'impulsion de courant ΔI peut être comprise entre 20 et 60 µs, la durée ΔTv de l'impulsion de tension ΔV peut être comprise entre 20 et 60 µs et le retard ΔT1 peut être compris entre 20 et 60 µs.

La durée ΔTi de l'impulsion de courant ΔI peut être de l'ordre de 50 µs, la durée ΔTv de l'impulsion de tension ΔV peut être de l'ordre de 25 µs et le retard ΔT1 peut être de l'ordre de 25 µs.

Les impulsions de tension ΔV peuvent être émises avec un retard ΔT2 après l'application des impulsions de courant ΔI. Le retard ΔT2 peut être compris entre 1 et 60 µs, et préférentiellement entre 10 et 30 µs.

La durée ΔTi de l'impulsion de courant ΔI peut être comprise entre 20 et 60 µs, la durée ΔTv de l'impulsion de tension ΔV peut être comprise entre 20 et 60 µs et le retard ΔT2 peut être compris entre 20 et 60 µs.

La durée ΔTi de l'impulsion de courant ΔI peut être de l'ordre de 50 µs, la durée ΔTv de l'impulsion de tension ΔV peut être de l'ordre de 25 µs et le retard ΔT2 peut être de l'ordre de 25 µs.

Dans des modes de réalisation de l'invention, les impulsions de tension ne sont émises qu'avec certaines impulsions de courant.

Les moyens de traitement du signal de réception peuvent être prévus pour analyser la variation temporelle de l'amplitude du signal électrique de réception du récepteur R.

Ces moyens d'exploitation peuvent être adaptés à comparer l'amplitude du signal à un niveau de référence. Le niveau de référence peut être fixé et déterminé par des moyens de référence. Les moyens de référence peuvent être agencés pour modifier le niveau de référence en fonction des valeurs du signal électrique de réception mesuré précédemment, ou en fonction des valeurs du signal électrique moyen mesuré précédemment. Les moyens de référence peuvent être agencés pour modifier le niveau de référence en fonction de la nature du risque d'incendie à surveiller.

La figure 4 montre un exemple de représentation temporelle de l'évolution du signal de réception Si, lorsqu'on applique une impulsion de courant ΔI à la source de lumière et une impulsion de tension ΔV entre les électrodes.

Comme il va de soi, et comme il résulte d'ailleurs déjà de ce qui précède, l'invention ne se limite nullement à ceux des modes d'applications et de réalisation qui ont été plus spécialement envisagés. Elle en embrasse au contraire toutes les variantes, notamment celles dans lesquelles le pinceau lumineux P issu d'une source de lumière, telle qu'une diode laser S d'émission, se propage le long de la zone de fort gradient créé près de l'électrode car on augmente ainsi considérablement la probabilité d'interaction des particules de fumées X avec le faisceau lumineux P.

Dans des variantes, les gradients de champ électromagnétique sont créés en focalisant des rayons lumineux, par exemple dans le domaine infra rouge, dans des zones très localisées à l'intérieur de la chambre, en utilisant des systèmes de focalisation connus en soi tels que des lentilles, des prismes ou des guides de lumière.

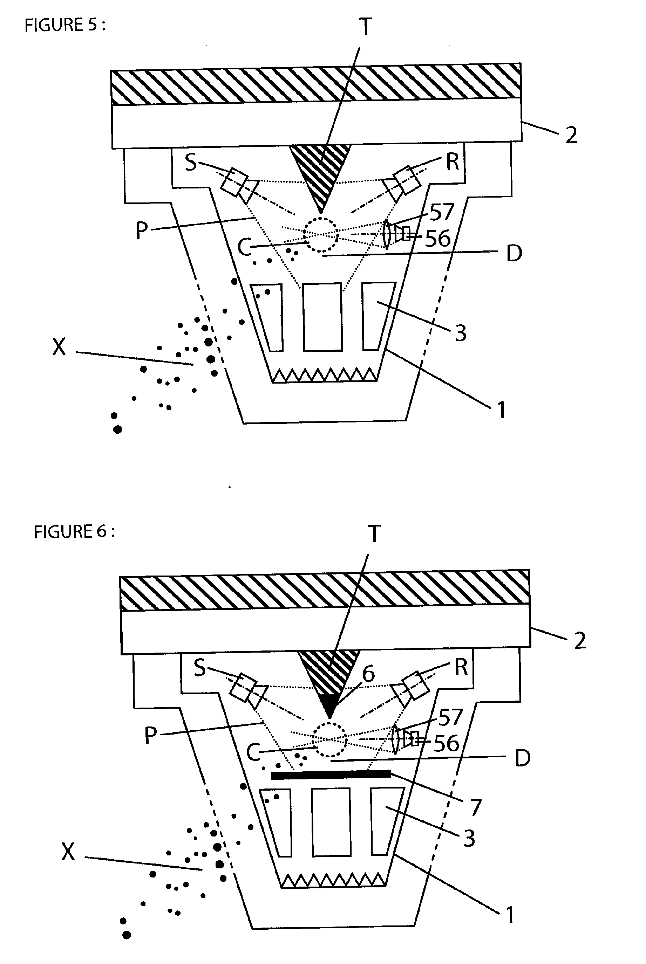

Ainsi selon un deuxième mode de réalisation de l'invention illustré en figure 5, les moyens de concentration sont constitués d'une source de rayons lumineux 56 dans le domaine infra rouge, par exemple une diode laser ou une diode électroluminescente, et une lentille 57 apte à focaliser les rayons lumineux dans la zone de détection D, pour créer une zone de concentration C. Cette zone correspond à la sous zone dite de fort gradient.

De cette manière, il est possible de concentrer les particules de fumée dans la région de concentration C prédéterminée du détecteur de telle sorte que le rayon lumineux en provenance de la source de lumière S et incident dans cette région de concentration C est diffusé vers le récepteur R par cette concentration élevée de particules de fumée, ce qui a l'effet d'augmenter la sensibilité du détecteur.

Bien entendu, dans des variantes, la source de lumière S est utilisée elle-même pour fournir ces rayons lumineux pour créer la zone de concentration C.

Dans encore des variantes, telle que celle illustrée dans le troisième mode de réalisation du dispositif objet de l'invention en figure 6, les moyens de concentration comprennent des électrodes 6 et 7 pour créer une région de concentration, ainsi qu'une source de rayons lumineux 56 dans le domaine infra rouge, et une lentille 57 pour améliorer la concentration de particules de fumée dans la région de concentration C.

Là encore, et comme il résulte d'ailleurs déjà de ce qui précède, ce troisième mode de réalisation ne se limite nullement à ceux des modes d'applications et de réalisations qui ont été plus spécialement envisagés. Ce mode de réalisation en embrasse, au contraire, toutes les variantes, notamment celles où le pinceau lumineux P est lui même utilisé pour créer les zones de fort gradient en mettant en oeuvre les moyens de focalisation décrits ci-dessus.

En suite de quoi, et quel que soit le mode de réalisation adopté, on obtient finalement un dispositif de détection optique dont la constitution et le fonctionnement résultent suffisamment de ce qui précède.

Ce dispositif présente, par rapport à ceux actuellement connus, de nombreux avantages et en particulier celui d'une sensibilité beaucoup plus grande aux fumées fines ou en faible concentration.

## Revendications

1. Un détecteur de fumée comprenant :
- une chambre (1) pourvue d'ouvertures (3) propres à permettre à la fumée de pénétrer à une zone de détection (D) dans la chambre ;
- une source de lumière (S) apte à émettre vers la zone de détection (D), un faisceau de rayons lumineux (P) ;
- un récepteur de lumière (R) sensible pour au moins une partie des longueurs d'onde des rayons lumineux émis par la source de lumière (S) et propre à transformer la réception de lumière en un signal électrique de réception ; le récepteur de lumière recevant, en présence de particules de fumée ou d'agrégats de particules de fumée dans la zone de détection (D), de la lumière en provenance de la zone de détection (D) ;
**caractérisé en ce qu'**il comporte, en outre :
- des moyens de concentration (6, 7, 16, 17, 26, 27, 36, 37, 46, 47, 56, 57) propres à créer, dans au moins une partie de la zone de détection (D), un champ électromagnétique non-homogène, apte, en présence de fumée, à polariser des particules de fumée qui pénètrent dans la zone de détection (D), le champ électromagnétique non-homogène ayant un gradient spatial de champ électromagnétique apte à exercer une force diélectrophorétique sur les particules de fumée de dimension comprise entre 0,01 µm et 10 µm apte à entraîner les particules de fumée polarisées dans une zone de concentration (C) dans la zone de détection (D).

2. Un détecteur selon la revendication 1, dans lequel les moyens de concentration (6, 7, 16, 17, 26, 27, 36, 37, 46, 47) comprennent deux électrodes (6, 7, 16, 17, 26, 27, 36, 37, 46, 47) propres à créer dans au moins une partie de la zone de détection (D) un champ électrique non-homogène, les électrodes étant agencées de façon à créer dans la zone de détection (D) un gradient spatial de champ électrique lorsqu'une différence de potentiel V, inférieure au seuil d'ionisation du gaz dans la chambre, est appliquée à ces électrodes.

3. Un détecteur selon la revendication 2, comportant en outre des moyens (10) pour appliquer la tension V aux bornes des électrodes, les moyens (10) étant propres à appliquer aux électrodes (6, 7, 16, 17, 26, 27, 36, 37, 46, 47) des impulsions de tension d'amplitude comprise entre 2 et 300V.

4. Un détecteur selon la revendication 3, dans lequel les impulsions de tension sont appliquées simultanément ou avec un retard après l'émission des impulsions de courant, le retard étant compris entre 1 et 60 µs.

5. Un détecteur selon l'une des revendications 2 ou 4, dans lequel une des électrodes (26, 36, 46) est constituée d'une pointe conductrice ou d'un segment d'un fil cylindrique conducteur ou d'une arête conductrice et l'autre électrode (27, 37, 47) est constituée d'une surface plane conductrice ou d'une portion d'arc d'une surface cylindrique.

6. Un détecteur selon l'une des revendications 2 à 5, dans lequel l'une des électrodes (7) est constituée par une partie de la chambre elle-même, et l'autre électrode (6) est située entre la source de lumière (S) et le récepteur (R) et empêche la vision directe entre la source de lumière (S) et le récepteur (R).

7. Un détecteur selon l'une des revendications 2 à 6, dans lequel l'une des électrodes est un fil conducteur placé parallèlement à l'axe de propagation de la lumière émise par la source de lumière (S).

8. Un détecteur selon l'une des revendications 2 à 7, dans lequel l'une des électrodes est constituée par l'arête d'un prisme, l'arête étant placée parallèlement à l'axe de propagation de la lumière émise par la source de lumière (S).

9. Un détecteur selon l'une des revendications 1 à 8, dans lequel les moyens de concentration comprennent un moyen de focalisation agencé pour focaliser des rayons lumineux, dans la zone de détection (D) pour engendrer des gradients de champ électromagnétique dans la zone de détection (D).

10. Un détecteur selon l'une des revendications 1 à 9, dans lequel la source de lumière est agencée de façon à permettre au faisceau de rayons lumineux de se propager le long de la zone de fort gradient de champ dans la zone de détection (D).

11. Un détecteur selon l'une des revendications 1 à 10, comprenant en outre des moyens d'alimentation de la source de lumière apte à fournir à la source de lumière des impulsions de courant d'amplitude ΔI et de durée ΔTi comprise entre 100 ns et 1 ms.

12. Un détecteur selon l'une des revendications 1 à 11, comprenant, en outre, des moyens de traitement du signal électrique de réception propres à comparer l'amplitude du signal (4) à un niveau de référence déterminé par des moyens de référence, les moyens de référence étant agencés pour modifier le niveau de référence en fonction des valeurs du signal électrique mesuré précédemment et/ou en fonction de la nature du risque d'incendie à surveiller.

13. Un détecteur selon l'une des revendications 1 à 12, dans lequel :
- le récepteur de lumière est agencé pour recevoir, en présence de particules de fumée ou d'agrégats de particules de fumée dans la zone de détection (D), de la lumière diffusée en provenance de la zone de détection (D) et
- la chambre (1) est agencée de façon à minimiser la pénétration de lumière extérieure dans la zone de détection et la source (S) et le récepteur (R) étant placés l'un par rapport à l'autre de manière propre à empêcher l'arrivée directe de la lumière émise par la source (s) sur le récepteur (R),

14. Un procédé de détection de fumée comprenant les étapes suivantes :
- l'émission d'une source de lumière (S) vers une zone de détection (D) dans une chambre (1) pourvue des ouvertures (3) propres à permettre à la fumée de pénétrer dans une zone de détection (D), d'un faisceau de rayons lumineux P ;
- la réception par un récepteur (R), en présence de particules de fumée ou d'agrégats de particules de fumée dans la zone de détection (D), de lumière en provenance de la zone de détection (D) ; et la transformation de la réception de lumière en un signal électrique de réception,
- la génération dans au moins une partie de la zone de détection (D) d'un champ électromagnétique non-homogène, pour, en présence de fumée, réaliser la polarisation par le champ électromagnétique des particules de fumée qui pénètrent dans la sous-zone de la zone de détection (D), le champ électromagnétique appliquant une force diélectrophorètique sur les particules de fumée de dimension comprise entre 0,01 µm et 10 µm pour entraîner des particules de fumée polarisées dans une zone de concentration (C) dans la zone de détection (D).

15. Un procédé selon la revendication 14, comportant, en outre, une étape d'agrégation des particules de fumée entre elles dans la zone de concentration pour former des particules de fumée plus grandes.

## Patentansprüche

1. Ein Rauchmelder umfassend:
- eine Kammer (1), die mit Öffnungen (3) versehen ist, die so ausgeführt sind, dass der Rauch in eine Erfassungszone (D) in der Kammer eindringen kann;
- eine Lichtquelle (S), die sich dazu eignet, ein Bündel an Lichtstrahlen (P) in die Erfassungszone (D) auszusenden;
- einen Lichtempfänger (R), der zumindest einen Teil der Wellenlängen der Lichtstrahlen wahrnehmen kann, die von der Lichtquelle (S) ausgesandt werden, und der so ausgeführt ist, dass er die Lichtaufnahme in ein elektrisches Empfangssignal umwandelt; der Lichtempfänger empfängt dabei bei Rauchpartikeln oder Aggregaten von Rauchpartikeln, die in der Erfassungszone (D) vorhanden sind, Licht aus der Erfassungszone (D);
**dadurch gekennzeichnet, dass** er darüber hinaus folgendes umfasst:
- Konzentrationsvorrichtungen (6, 7, 16, 17, 26, 27, 36, 37, 46, 47, 56, 57), die so ausgeführt sind, dass sie in zumindest einem Abschnitt der Erfassungszone (D) ein nicht homogenes elektromagnetisches Feld bilden, das in der Lage ist, bei vorhandenem Rauch Rauchpartikel zu polarisieren, die in die Erfassungszone (D) eindringen, wobei das nicht homogene elektromagnetisches Feld einen räumlichen Gradienten des elektromagnetischen Feldes aufweist, der in der Lage ist, eine dielektrophoretische Kraft auf die Rauchpartikel in der Größe zwischen 0,01 µm und 10 µm auszuüben, die in der Lage ist, die polarisierten Rauchpartikel in eine Konzentrationszone (C) in der Erfassungszone (D) anzutreiben.

2. Rauchmelder nach Anspruch 1**,** bei dem die Konzentrationsvorrichtungen (6, 7, 16, 17, 26, 27, 36, 37, 46, 47) zwei Elektroden (6, 7, 16, 17, 26, 27, 36, 37, 46, 47) umfassen, die so ausgeführt sind, um in zumindest einem Abschnitt der Erfassungszone (D) ein nicht homogenes elektrisches Feld zu bilden, und die Elektroden angeordnet sind, um in der Erfassungszone (D) einen räumlichen Gradienten des elektromagnetischen Feldes zu bilden, wenn ein Potenzialunterschied (V) auf diese Elektroden angewandt wird, der kleiner dem Grenzwert der Gasionisierung in der Kammer ist.

3. Rauchmelder nach Anspruch 2, der darüber hinaus Vorrichtungen (10) enthält, um die Spannung V an die Klemmen der Elektroden anzulegen, wobei die Vorrichtungen (10) so ausgeführt sind, um Spannungsimpulse an den Elektroden (6, 7, 16, 17, 26, 27, 36, 37, 46, 47) anzulegen, deren Amplitude zwischen 2 und 300V liegt.

4. Rauchmelder nach Anspruch 3, bei dem die Spannungsimpulse gleichzeitig oder mit einer Verzögerung nach der Aussendung der Stromimpulse angelegt werden, wobei die Verzögerung zwischen 1 und 60 µs liegt.

5. Rauchmelder nach einem der Ansprüche 2 oder 4, bei dem eine der Elektroden (26, 36, 46) aus einer leitenden Spitze oder einem Segment eines zylindrischen leitenden Kabels gebildet wird, oder aus einer leitenden Kante, und die andere Elektrode (27, 37, 47) aus einer ebenen leitenden Fläche oder einem Bogenabschnitt einer zylindrischen Oberfläche gebildet wird.

6. Rauchmelder nach einem der Ansprüche 2 bis 5, bei dem die eine der Elektroden (7) aus einem Abschnitt der Kammer selbst gebildet wird, und die andere Elektrode (6) zwischen der Lichtquelle (S) und dem Empfänger (R) angeordnet ist, und den direkten Blick zwischen der Lichtquelle (S) und dem Empfänger (R) verhindert.

7. Rauchmelder nach einem der Ansprüche 2 bis 6, bei dem die eine der Elektroden ein leitendes Kabel ist, das parallel zur Fortpflanzungsachse des Lichts angeordnet ist, das von der Lichtquelle (S) ausgesandt wird.

8. Rauchmelder nach einem der Ansprüche 2 bis 7, bei dem die eine der Elektroden aus der Kante eines Prismas besteht, wobei die Kante parallel zur Ausbreitungsachse des Lichts angeordnet ist, das von der Lichtquelle (S) ausgesandt wird.

9. Rauchmelder nach einem der Ansprüche 1 bis 8, bei dem die Konzentrationsvorrichtungen eine Fokalisierungsvorrichtung umfassen, die zum Fokalisieren der Lichtstrahlen in der Erfassungszone (D) angeordnet sind, um in der Erfassungszone (D) Gradienten des elektromagnetischen Feldes zu bilden.

10. Rauchmelder nach einem der Ansprüche 1 bis 9, bei dem die Lichtquelle so angeordnet ist, dass sich das Bündel an Lichtstrahlen in der Erfassungszone (D) entlang der Zone des starken Feldgradienten ausbreiten kann.

11. Rauchmelder nach einem der Ansprüche 1 bis 10, der darüber hinaus Vorrichtungen zur Versorgung der Lichtquelle umfasst, die sich dazu eignen, der Lichtquelle Stromimpulse mit einer Amplitude von ΔI und einer Dauer von ΔTi zu liefern, die zwischen 100 ns und 1 ms liegt.

12. Rauchmelder nach einem der Ansprüche 1 bis 11, der darüber hinaus Vorrichtungen zur Bearbeitung des elektrischen Empfangssignals umfasst, die so ausgeführt sind, um die Amplitude des Signals (4) mit einem Bezugsniveau zu vergleichen, das von Bezugsvorrichtungen festgelegt wird, wobei die Bezugsvorrichtungen angeordnet sind, um das Bezugsniveau in Abhängigkeit von den Werten des elektrischen Signals, das zuvor gemessen wurde und/ oder in Abhängigkeit von der Art der Brandgefahr zu verändern, die es zu überwachen gilt.

13. Rauchmelder nach einem der Ansprüche 1 bis 12, in dem
- der Lichtempfänger angeordnet ist, um bei vorhandenen Rauchpartikeln oder Aggregaten von Rauchpartikeln in der Erfassungszone (D) Licht zu empfangen, das aus der Erfassungszone (D) ausgestrahlt wird, und
- die Kammer (1) angeordnet ist, um das Eindringen von Außenlicht in die Erfassungszone zu minimieren, und die Quelle (S) und der Empfänger (R) so zueinander platziert sind, dass sie sich dazu eignen, das direkte Eintreffen des von der Quelle (S) ausgestrahlten Lichts auf den Empfänger (R) zu verhindern,

14. Verfahren zum Erfassen von Rauch, das die folgenden Schritte umfasst:
- die Aussendung einer Lichtquelle (S) in eine Erfassungszone (D) in einer Kammer (1), die mit Öffnungen (3) versehen ist, die so ausgeführt sind, dass der Rauch in eine Erfassungszone (D) eines Bündels an Lichtstrahlen P eindringen kann;
- den Empfang des Lichts aus der Erfassungszone (D) durch einen Empfänger (R) bei Vorhandensein von Rauchpartikeln oder Aggregaten von Rauchpartikeln in der Erfassungszone (D); und die Umwandlung des Lichtempfangs in ein elektrisches Empfangssignal,
- die Bildung eines nicht homogenen elektromagnetischen Feldes in zumindest einem Abschnitt der Erfassungszone (D), um bei vorhandenem Rauch Rauchpartikel durch das elektromagnetische Feld zu polarisieren, die in die Unterzone der Erfassungszone (D) eindringen, wobei das elektromagnetische Feld eine dielektrophoretische Kraft auf die Rauchpartikel in der Größe zwischen 0,01 µm und 10 µm ausübt, um die polarisierten auchpartikel in eine Konzentrationszone (C) in der Erfassungszone (D) anzutreiben.

15. Verfahren nach Anspruch 14, das darüber hinaus einen Schritt zur Aggregation der Rauchpartikel untereinander in der Konzentrationszone umfasst, um größere auchpartikel zu bilden.

## Claims

1. Smoke detector comprising:
- a chamber (1) provided with apertures (3) able to allow the smoke to enter a detection area (D) in the chamber;
- a light source (S) able to emit a beam of light rays (P) towards the detection area (D);
- a light receiver (R) sensitive to at least one portion of the wavelengths of the light rays emitted by the light source (S) and able to transform the reception of light into an electrical reception signal; the light receiver receiving, in the presence of smoke particles or of smoke particle aggregates in the detection area (D), light coming from the detection area (D);
**characterized in that** it comprises, in addition:
- concentration means (6, 7, 16, 17, 26, 27, 36, 37, 46, 47, 56, 57) able to create a non-uniform electromagnetic field in at least one portion of the detection area (D), which, in the presence of smoke, can polarize smoke particles entering the detection area (D), the non-uniform electromagnetic field having a spatial electromagnetic field gradient able to exert a dielectrophoretic force on the smoke particles of between 0.01 µm and 10 µm in size that is able to drive the polarized smoke particles into a concentration area (C) in the detection area (D).

2. Detector according to claim 1, wherein the concentration means (6, 7, 16, 17, 26, 27, 36, 37, 46, 47) comprise two electrodes (6, 7, 16, 17, 26, 27, 36, 37, 46, 47) able to create a non-uniform electric field in at least one portion of the detection area (D), the electrodes being arranged so as to create a spatial electric field gradient in the detection area (D) when a potential difference V, lower than the ionization threshold of the gas in the chamber, is applied to these electrodes.

3. Detector according to claim 2, comprising in addition means (10) of applying the voltage V to the terminals of the electrodes, the means (10) being able to apply voltage pulses of between 2 and 300V in amplitude to the electrodes (6, 7, 16, 17, 26, 27,36,37,46,47).

4. Detector according to claim 3, wherein the voltage pulses are applied at the same time as or with a delay after the current pulses are emitted, the delay being between 1 and 60 µs.

5. Detector according to one of claims 2 or 4, wherein one of the electrodes (26, 36, 46) consists of a conductive tip, a segment of a cylindrical conductive wire or a conductive edge and the other electrode (27, 37, 47) consists of a flat conductive surface or a portion of an arc of a cylindrical surface.

6. Detector according to one of claims 2 to 5, wherein a portion of the chamber itself constitutes one of the electrodes (7), and the other electrode (6) is located between the light source (S) and the receiver (R) and prevents the receiver (R) from having a direct view of the light source (S).

7. Detector according to one of claims 2 to 6, wherein one of the electrodes is a conductive wire placed parallel to the propagation axis of the light emitted by the light source (S).

8. Detector according to one of claims 2 to 7, wherein one of the electrodes is formed by the edge of a prism, the edge being placed parallel to the propagation axis of the light emitted by the light source (S).

9. Detector according to one of claims 1 to 8, wherein the concentration means comprise a focusing means arranged to focus light rays in the detection area (D), to generate electromagnetic field gradients in the detection area (D).

10. Detector according to one of claims 1 to 9, wherein the light source is arranged so as to allow the beam of light rays to be propagated along the high field gradient area in the detection area (D).

11. Detector according to one of claims 1 to 10, comprising in addition means of powering the light source able to supply current pulses of amplitude ΔI and of duration ΔTi between 100 ns and 1 ms to the light source.

12. Detector according to one of claims 1 to 11, comprising in addition the electrical reception signal processing means are able to compare the amplitude of the signal (4) to a reference level determined by reference means, the reference means being arranged to alter the reference level according to values of the electrical signal measured previously and/or according to the nature of the fire risk to be monitored.

13. Detector according to one of claims 1 to 12, wherein
- the light receiver is arranged to receive, in the presence of smoke particles or of smoke particle aggregates in the detection area (D), diffused light coming from the detection area (D) and
- the chamber (1) is arranged so as to minimize the penetration of outside light into the detection area and the source (S) and the receiver (R) being placed relative to each other so as to able to prevent the light emitted by the source (S) from reaching the receiver (R) directly.

14. Method for detecting smoke, comprising the following steps:
- emission of a beam of light rays P from a light source (S) towards a detection area (D) in a chamber (1) provided with apertures (3) able to allow the smoke to enter a detection area (D);
- reception by a receiver (R), in the presence of smoke particles or of smoke particle aggregates in the detection area (D), of light coming from the detection area (D); and the transformation of the reception of light into an electrical reception signal,
- the generation of a non-uniform electromagnetic field in at least one portion of the detection area (D), so that, in the presence of smoke, smoke particles entering the sub-area of the detection area (D) are polarized by the electromagnetic field, the electromagnetic field applying a dielectrophoretic force on the smoke particles of between 0.01 µm and 10 µm in size so as to drive the polarized smoke particles into a concentration area (C) in the detection area (D).

15. Method according to claim 14, comprising in addition a step of aggregating smoke particles to each other in the concentration area so as to form larger smoke particles.
